# EUROPEAN PATENT APPLICATION

(11) **EP 0 821 957 A2**
(43) Date of publication of application: **04.02.1998**
(21) Application number: 97305592.4
(22) Date of filing: 25.07.1997
(51) Int. Cl.: A61K 31/44

(54) **Use of 3-(4-hexyloxy-1,2,5-thiadiazol-3-yl)-1,2,5,6-tetrahydro-1-methylpyridine (xanomeline) for treating substance abuse**

(30) Priority: 01.08.1996 US 22901 P
(71) Applicant: ELI LILLY AND COMPANY, Indianapolis, Indiana 46285 (US)
(72) Inventor: Rasmussen, Kurt, Fishers, Indiana 46038 (US)
(74) Representative: Hudson, Christopher Mark

(57) **Abstract**

The present invention provides a method for treating substance abuse in humans using a compound of the Formula I

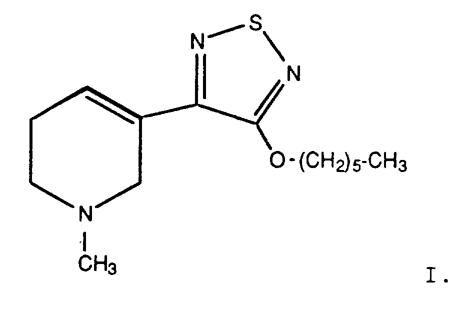

## Description

This invention provides a method for using 3-(4-hexyloxy-1,2,5-thiadiazol-3-yl)-1,2,5,6-tetrahydro-1-methylpyridine, (hereinafter referred as "xanomeline"), for the treatment of dependence on a controlled substance.

As long as history has been recorded, every society has used drugs that alter mood, thought, and feeling. In addition, pharmacological advances sometimes have been paralleled by physical as well as unfortunate behavioral dependence on agents initially consumed for therapeutic purposes. Therefore, the alleviation and eventual withdrawal from undesired physical and psychological dependence and tolerance of a substance has been a challenge throughout history. Although there are some treatments availble for such withdrawal from addictive and/or mind altering substances are available, there is a great need for safer and more effective treatments.

Alcohol abuse is a particularly prevelant form of substance abuse contemplated by the present invention. The lifetime prevalence of alcohol abuse and alcohol addiction is 5% to 10% for men and 3% to 5% for women. Goodman & Gillman's The Pharmacological Basis of Therapeutics, 562 (9th Ed. 1996, McGraw-Hill, New York). Experience with alcohol can produce greater tolerance such that extremely high blood levels can be found in alcoholics who do not appear grossly intoxicated. In such circumstances, the lethal dose does not increase proportionately, and thus the margin of safety is decreased.

In addition to the dangers associated with a narrowed margin of safety, the alcoholic patient inevitably develops a state of physical dependence. The chronic use of alcohol is associated with the development of depression, and the risk of suicide among alcoholics is one of the highest of any diagnostic catagory. Further, alcohol is toxic to many organ systems. As a result, the medical complications of alcohol abuse and addiction include liver disease, cardiovascular disease, endocrine and gastrointestinal effects, and malnutrition in addition to psychological dysfunctions.

Unfortunately, withdrawal from alcohol can require hospitalization and close medical evaluation to avoid serious physical consequences and symptoms. Most medications currently used to alleviate and/or facilitate the withdrawal from alcohol are not as effective as desired. There are widespread efforts to develop new pharmacological agents the treatment of alcohol addiction and abuse. see, Goodman & Gillman's The Pharmacological Basis of Therapeutics, 563 (9th Ed. 1996, McGraw-Hill, New York).

It would be particularly desired to provide an effective treatment for these forms of addiction that could minimize hospitalization or institutionalization of a patient. The treatment must be non-addictive and provide a favorable side effect profile. Applicants believe that xanomeline could fulfill this need.

Applicants have discovered that 3-(4-hexyloxy-1,2,5-thiadiazol-3-yl)-1,2,5,6-tetrahydro-1-methylpyridine, thought to be a muscarinic agonist, can be useful for treating addiction to a controlled substance and withdrawal from such substance. The present invention relates to a method of treating addiction to a controlled substance and withdrawal from such substance. More specifically, the invention provides a method of treating substance abuse in humans using xanomeline.

As noted hereinbefore, the compounds employed in the method of the present invention are known. Methods of preparing the compounds, as well as pharmaceutical formulations containing the compounds, are taught by Sauerberg in U.S. Pat. No. 5,043,345 (hereinafter refered to as the "'345 patent") herein incorporated by reference. The '345 patent teaches that xanomeline can be useful for treating Alzheimer's Disease and as stimulants of the cognitive function of the forebrain and hippocampus of mammanls. Applicants have discovered that xanomeline can be useful for the treatment of substance abuse and withdrawal from a controlled substance. Xanomeline may address the long felt need for treatments having an acceptable safety profile and provide effective relief to the patient suffering from an addiction to a controlled substance. Further, Xanomeline may suppress the occurance of relapse abuse.

The presently claimed invention provides a method for treating a condition which is a response produced by cessation and withdrawal from the abuse of alcohol, comprising administering an effective amount of Formula I: 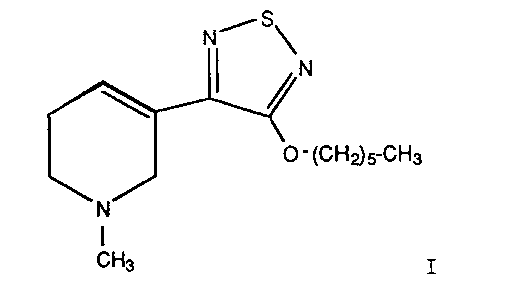 or
a pharmaceutically acceptable salt or solvate thereof to a patient in need of such treatment.

This invention further provides a method for treating alcoholism comprising administering an effective amount of xanomeline or a pharmaceutically acceptable salt thereof to a patient in need of such treatment.

This invention provides a method for treating physical dependence on alcohol comprising administering an effective amount of xanomeline or a pharmaceutically acceptable salt thereof to a patient in need of or desiring such treatment.

Finally, the present invention provides a method for treating alcohol withdrawal syndrome comprising administering an effective amount of xanomeline or a pharmaceutically acceptable salt thereof to a patient in need of such treatment.

The presently claimed invention provides a method for treating a condition which is a response produced by cessation and withdrawal from the abuse of a substance which is physically and/or psychologically addictive, comprising administering an effective amount of Formula I: 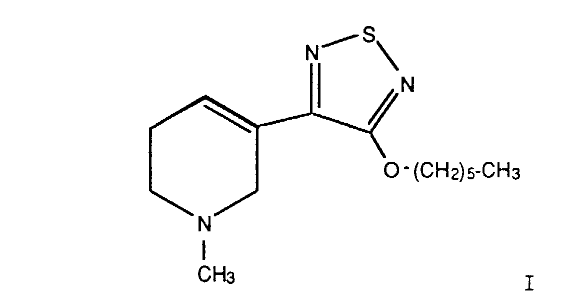 or
a pharmaceutically acceptable salt or solvate thereof to a patient in need of such treatment.

This invention further provides a method for treating substance abuse comprising administering an effective amount of xanomeline or a pharmaceutically acceptable salt thereof to a patient in need of such treatment.

This invention provides a method for treating dependence on a mind, thought or mood altering substance comprising administering an effective amount of xanomeline or a pharmaceutically acceptable salt thereof to a patient in need of or desiring such treatment.

Said substances include, but are in no way limited to opioids, anxiolytic and hypnotic drugs, cocaine, psychedelic agents, marijuana, amphetamines, hallucinogens, phencyclidine, and benzodiazepines.

The term "effective amount", as used herein, represents an amount of compound necessary to prevent or treat a human susceptible to or suffering from substance addiction following administration to such human. The active compound is effective over a wide dosage range. For example, dosages per day will normally fall within the range of about 0.005 to about 500 mg/kg of body weight. In the treatment of adult humans, the range of about 0.05 to about 100 mg/kg, in single or divided doses, is preferred. However, it will be understood that the amount of the compound actually administered will be determined by a physician, in the light of the relevant circumstances including the condition to be treated, the choice of compound to be administered, the age, weight, and response of the individual patient, the severity of the patient's symptoms, and the chosen route of administration, and therefore the above dosage ranges are not intended to limit the scope of the invention in any way. While the present compound may be administered orally to humans susceptible to or suffering from substance abuse, the compound is particularly well suited to be administered transdermally. When the compound is deleivered transdermally, it is preferred that the effective amount is from about 10mg to about 100mg per day delivery of base compound. It is especially preferred that such patch delivers an effective amount for about one to seven days.

The compound may further be delivered by a variety of other pharmaceutically accepted routes including, but in no way limited to parenterally, subcutaneous, intranasal, intramuscular and intravenous routes. Such formulations may be designed to provide delayed or controlled release using formulation techniques which are known in the art.

As used herein the term "substance abuse" shall refer to the undesired physical and/or psychological dependence on a drug. The term shall most preferedly refer to dependence on a substance selected from the group consisting of opioids, anxiolytic and hypnotic drugs, cocaine, psychedelic agents, marijuana, amphetamines, hallucinogens, phencyclidine, and benzodiazepines.
Additional compounds producing a physical and/or psychological dependence are contemplated as well.

The term "mind, thought or mood altering substance" shall refer to a substance which alters the though process. For purposes of this invention, such substance additionally produces an undesired physical and/or psychological dependence or tolerance. Said substances shall include, but are in no way limited to a compound selected from the group consisting of opioids, anxiolytic and hypnotic drugs, cocaine, psychedelic agents, marijuana, amphetamines, hallucinogens, phencyclidine, and benzodiazepines.

As used herein the term "treating" includes prophylaxis of a physical and/or mental condition or amelioration or elimination of the developed physical and/or mental condition once it has been established or alleviation of the characteristic symptoms of such condition. The term "treating dependence" shall mean that xanomeline will be used to suppress or alleviate the desire of a patient who has suffered from substance abuse to relapse.

As used herein, the term "substance abuse" shall refer to a condition wherein the patient suffers physical consequences attributable to the ingestion of a substance and is unable or unwilling to cease such substance consumption and treatment to facilitate withdrawal from said substance is desired.

As used herein, the term "alcohol abuse" shall refer to a condition wherein the patient suffers physical consequences attributable to the ingestion of alcohol and is unable or unwilling to cease such alcohol consumption and treatment to facilitate withdrawal from alcohol consumption is desired.

As used herein, the term "alcoholism" or "physical dependence on alcohol" shall refer to a condition resulting from excessive consumption of alcohol. The patient suffering from alcoholism is identified by severe dependence or addiction and a cumulative pattern of behaviors associated with drinking. Frequent intoxication is obvious and destructive; it interferes with the individual's ability to socialize and to work. Many alcoholics experience marriage failure, work absenteeism which may lead to being fired. Alcoholics may seek medical treatment for their drinking, they may suffer physical injury associated with their drinking, and they may be apprehended while driving intoxicated. Eventually, the alcoholic may be arrested for drunkedness and/or hospitalized for delirium tremens or cirrhosis of the liver.

As used herein, "alcohol withdrawal" shall refer to a characteristic withdrawal syndrome that develops after the cessation of (or reduction in) heavy and prolonged alcohol use. Alcohol withdrawal syndrome is summarized in Goodman Gillman's The Pharmacological Basis of Therapeutics, 563 (9th Ed. 1996, McGraw-Hill, New York). To further clarify, Alcohol Withdrawal is characterized in the DSM-IV-R. Diagnostic and Statistical Manual of Mental Disorders, Revised, 3rd Ed. (1994) as catagory 291.8. The DSM-IV-R was prepared by the Task Force on Nomenclature and Statistics of the American Psychiatric Association, and provides clear descriptions of diagnostic catagories. The skilled artisan will recognize that there are alternative nomenclatures, nosologies, and classification systems for pathologic psychological conditions and that these systems evolve with medical scientific progress.

The discontinuation or a reduction of excessive alcohol consumption typically results in alcohol withdrawal syndrome. Alcohol withdrawal syndrome is a continuum of symptoms and signs which accompany alcohol withdrawal, usually beginning 12 to 48 hours after a significant decrease in alcohol intake. For example, such symptoms may include tremor, weakness, sweating, gastrointestinal symptoms, increasing confusion, poor sleep, and/or severe depression. These symptoms often cause clinically significant distress or impairment in social, occupational, or other important areas of functioning. The present invention is most preferably used to alleviate symptoms attributed to alcohol withdrawal when such symptoms are not due to a general medical condition and are not better accounted for by a mental disorder.

The method of the present invention is preferably administered in connection with an educational and/or behavioral modification program to ensure continued abstinence from alcohol. The method of the present invention is also highly beneficial to such programs by alleviating the suffering experienced from the alcohol withdrawal over the course of such programs. Therefore, the programs can be more effective by focusing on educational and behavioral modification goals, further reducing the incidence of program non-completion.

The compounds employed in the invention are not believed to act via the GABA/benzodiazepine, serotonin, or dopamine receptor systems in humans. Rather, the activity of the present compound as a treatment for substance abuse is believed to be based upon modulation of muscarinic cholinergic receptors. However, the mechanism by which the present compounds function is not necessarily the mechanism stated *supra.,* and the present invention is not limited by any mode of operation.

Xanomeline has been studied using accepted pharmacological methods such as oxotremorine-M verses N-methylscopolamine binding studies (Freedman et al. Br. J. Pharmacology, **93**:437-445 (1988). Xanomeline inhibited the binding of ³H-oxotremorine-M with an inhibition costant (Kᵢ) of 2nM. The binding of the muscarinic ml antagonist ligand, ³H-pirenzepine, to ml receptors in hippocampus and ³H-quinuclidinyl benzilate to m2 receptors in brain stem was inhibited with Kᵢ values of 5 and 24 nM, respectively.

Muscarinic agonists stimulate the formation of cAMP up to 10 fold in CHO m4 cells treated with pertussisi toxin and the pharmacology is consistent with the mediation by m4 receptors. Eckols K. Soc. Neurosci Abstr., **21**:2040 (1995). In this assay, xanomeline efficaciously and potently stimulated the formation of cAMP. Such studies suggest that xanomeline predominantly activates ml and m4 receptors.

The usefulness of the compound for treating a condition resulting from cessation and withdrawal from the use of a dependence producing substance can be supported by the following studies as described.

### I. Auditory Startle Response.

Male Long Evans rats (Harlan Sprague Dawley) are individually housed in a controlled environment on a 12 hour light-dark cycle. The rats are given free access to food and water. All treatment groups contain from 8 to 10 rats.

The rats are anesthetized with halothane and Alzet osmotic minipumps (Alza Corporation, Palo Alto, California) are implanted subcutaneously. A dependence producing substance, such as an opioid, is provided in physiological saline. Pumps are filled with opioid (6 mg/kg base/day) or the appropriate vehicle. Twelve days following implantation of pumps, rats are anesthetized with halothane and the pumps are removed. (The study is also conducted with other dependence producing substances such as marijuana, and so on.)

The auditory Startle Response is observed.

The sensory motor reactions [auditory startle response (peak amplitude, Vₘₐₓ)] of individual rats are recorded using San Diego Instruments startle chambers (San Diego, Calif.). Startle sessions consist of a 5 minute adaptation period at background noise level of 70 +/- 2dBA immediately followed by 25 presentations of auditory stimuli (120 +/-3 dBA noise, 50 ms duration) presented at 8 second intervals. Peak startle amplitudes are averaged for all 25 presentations of stimuli for each session. Auditory startle responding is evaluated daily at 24 hour intervals on days 1-4 following substance withdrawal.

Xanomeline can be prepared as described in the '345 patent.

The following Examples are studies to establish the usefulness of the named compounds for treating substance abuse.

### Example 1

### Human Clinical Trials

The activity of 3-(4-hexyloxy-1,2,5-thiadiazol-3-yl)-1,2,5,6-tetrahydro-1-methylpyridine for treating or alleviating substance abuse can be demonstrated by human clinical trials. The study was designed as a double-blind, parallel, placebo-controlled multicenter trial. The subjects were randomized into four groups, placebo and 25, 50, and 75 mg tid of test compound. The dosages were administered orally with food. Subjects were observed at four visits to provide baseline measurements. Visits 5-33 served as the treatment phase for the study.

During the visits, subjects are observed for signs of agitation, mood swings, tremor, delirium, social withdrawal, and concentration abilities.

Treatment groups are compared with respect to the number and percent of subjects who ever had the symptom during the double-blind portion of the study (visits 5 through 33), at a severity that was worse than during the baseline visits (1 through 4).

## Claims

1. A method for treating substance abuse comprising administering to a mammal in need of such treatment, an effective amount of a compound of Formula I: 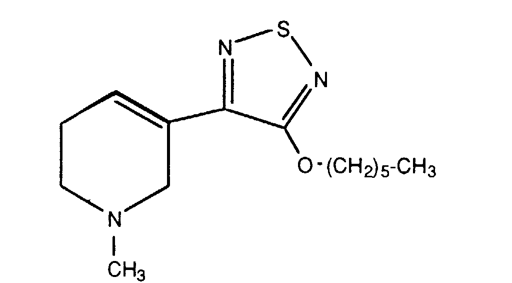 or a pharmaceutically acceptable salt thereof.

2. A method of **Claim 1** wherein the substance abuse is physical dependence on a compound selected from the group consisting of alcohol, opioids, anxiolytic and hypnotic drugs, cocaine, psychedelic agents, marijuana, amphetamines, hallucinogens, phencyclidine, and benzodiazepines.

3. A method of **Claim 2** wherein the compound is selected from the group consisting of anxiolytic and hypnotic drugs, cocaine, psychedelic agents, amphetamines, hallucinogens, phencyclidine, and benzodiazepines.

4. A method of **Claim 2** wherein the physcial dependence is on an opioid.

5. A method of **Claim 1** wherein the effective amount is from 1 mg/kg to about 100 mg/kg per day.

6. A method of **Claim 5** wherein the effective amount is from about 10 mg/kg to about 100 mg/kg per day.

7. A method of **Claim 1** wherein the effective amount is delivered using a transdermal patch.

8. A method of **Claim 5** wherein the transdermal patch delivers from about 10 to about 100 mg of base compound per day.

9. A method of **Claim 8** wherein the transdermal patch delivers an effective amount for one (1) to seven (7) days.

10. A method for treating a condition which is a response produced by cessation and withdrawal from the use of a physcial or psycholocally addictive substance,
comprising administering an effective amount of a compound of Formula I: 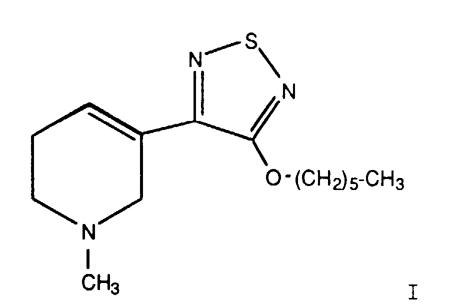 or a pharmaceutically acceptable salt thereof to a patient in need of such treatment.

11. A method of **Claim 10** wherein the response is substance withdrawal syndrome.

12. A method for treating alcohol abuse comprising administering to a mammal in need of such treatment, an effective amount a compound of Formula I: 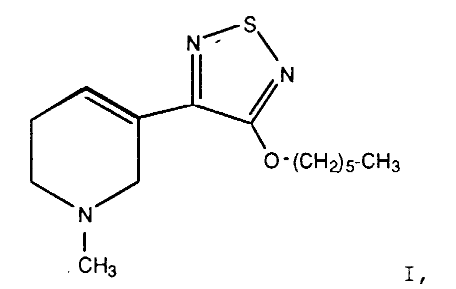 or a pharmaceutically acceptable salt thereof.

13. A method of **Claim 12** wherein the alcohol abuse is physical dependence on alcohol.

14. A method of **Claim 13** wherein the physcial dependence on alcohol is alcoholism.

15. A method for treating a condition which is a response produced by cessation and withdrawal from the abuse of alcohol, comprising administering an effective amount of a compound of Formula I: 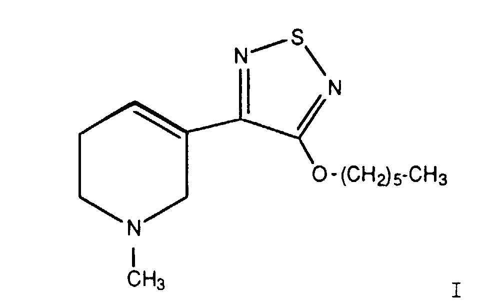 or a pharmaceutically acceptable salt thereof to a patient in need of such treatment.

16. A method of **Claim 15** wherein the response is alcohol withdrawal syndrome.

17. A method of **Claim 15** wherein the condition is relapse abuse.

18. Use of a compound of Formula I: 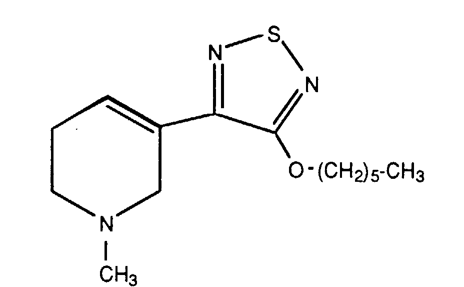 or a pharmaceutically acceptable salt thereof for the manufacture of a medicament for treating substance abuse.

19. A use of **Claim 18** wherein the substance abuse is physical dependence on a compound selected from the group consisting of alcohol, opioids, anxiolytic and hypnotic drugs, cocaine, psychedelic agents, marijuana, amphetamines, hallucinogens, phencyclidine, and benzodiazepines.

20. A use of **Claim 18** wherein the compound of Formula I is delivered using a transdermal patch.

21. A use of **Claim 20** wherein the transdermal patch delivers from about 10 to about 100 mg of base compound per day.

22. A use of **Claim 20** wherein the transdermal patch delivers an effective amount for one (1) to seven (7) days.

23. A use of a compound of Formula I: 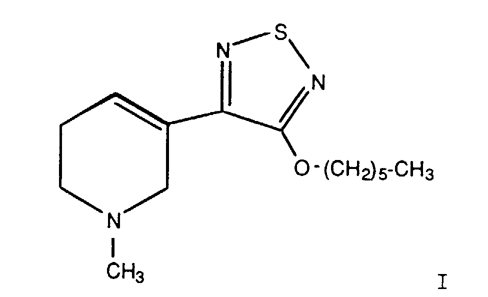 or a pharmaceutically acceptable salt for the manufacture of a medicament for treating a condition which is a response produced by cessation and withdrawal from the use of a physical or psychologically addictive substance

24. A use of **Claim 23** wherein the response is substance withdrawal syndrome.

25. A use of a compound of Formula I: 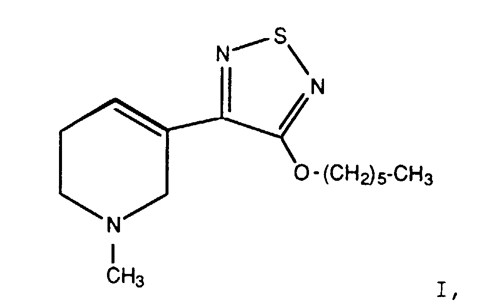 or a pharmaceutically acceptable salt thereof for the manufacture of a medicament for the treatment of alcohol abuse.

26. A use of **Claim 25** wherein the alcohol abuse is physical dependence on alcohol.

27. Use of a compound of Formula I: 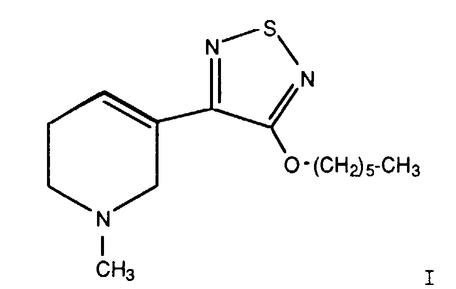 or a pharmaceutically acceptable salt thereof for the manufacture of a medicament for the treatment of substance abuse.
